(19) Europäisches Patentamt
European Patent Office
Office européen des brevets

(11) Veröffentlichungsnummer: **0 114 603**
**B1**

(12) # EUROPÄISCHE PATENTSCHRIFT

(45) Veröffentlichungstag der Patentschrift: **08.06.88**

(21) Anmeldenummer: **84100128.2**

(22) Anmeldetag: **09.01.84**

(51) Int. Cl.⁴: **C 07 D 401/06,** A 61 K 31/445, A 61 K 31/44 // (C07D401/06, 211:70, 209:14)

(54) **Indolderivate.**

(30) Priorität: **20.01.83 DE 3301758**

(43) Veröffentlichungstag der Anmeldung: **01.08.84 Patentblatt 84/31**

(45) Bekanntmachung des Hinweises auf die Patenterteilung: **08.06.88 Patentblatt 88/23**

(84) Benannte Vertragsstaaten: **AT BE CH DE FR GB IT LI NL SE**

(56) Entgegenhaltungen:
**EP-A-0 007 399**
**EP-A-0 077 607**
**CH-A-528 525**
**DE-A-1 620 750**
**DE-A-2 708 913**
**US-A-4 160 862**

**Die Akte enthält technische Angaben, die nach dem Eingang der Anmeldung eingereicht wurden und die nicht in dieser Patentschrift enthalten sind.**

(73) Patentinhaber: **Merck Patent Gesellschaft mit beschränkter Haftung, Frankfurter Strasse 250, D-6100 Darmstadt (DE)**

(72) Erfinder: **Hausberg, Hans- Heinrich, Dr., Odenwaldstrasse 30, D-6105 Ober- Ramstadt (DE)**
Erfinder: **Böttcher, Henning, Dr., Soderstrasse 95, D-6100 Darmstadt (DE)**
Erfinder: **Gottschlich, Rudolf, Dr., Buchenweg 1, D-6107 Reinheim (DE)**
Erfinder: **Seyfried, Christoph, Dr., Mathildenstrasse 6, D-6104 Seeheim- Jugenheim (DE)**
Erfinder: **Minck, Klaus- Otto, Dr., Büchestrasse 8, D-6105 Ober- Ramstadt (DE)**

**EP 0 114 603 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die Erfindung betrifft neue Indolderivate der allgemeinen Formel

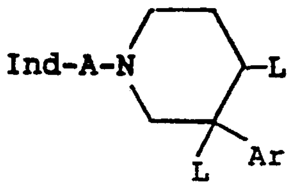

I

worin

Ind einen indol-3-ylrest, der ein- oder zweifach durch Alkyl, O-Alkyl, S-Alkyl, SO-Alkyl, $SO_2$-Alkyl, OH, F, Cl, Br, $CF_3$ und/oder CN oder durch eine Methylendioxygruppe substituiert sein kann,

A $-(CH_2)_n-$,

n 3, 4 oder 5

Ar eine Hydroxyphenylgruppe und die beiden Reste L jeweils H oder zusammen eine C-C-Bindung bedeuten und

worin die Alkylgruppen jeweils 1 - 4 C-Atome besitzen,

sowie deren physiologisch unbedenkliche Säureadditionssalze.

Nachstehend wird der Einfachheit halber die Gruppe

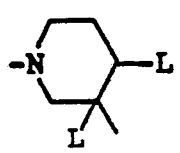

als "Q" bezeichnet, so daß die Formel 1 auch in der Form Ind-A-Q-Ar geschrieben werden kann.

In der EP-A-7 399 und der EP-A-77 607 sind Verbindungen ähnlicher Struktur und mit ähnlichen pharmakologischen Wirkungen beschrieben.

Der Erfindung lag die Aufgabe zugrunde, neue Verbindungen aufzufinden, die zur Herstellung von Arzneimitteln verwendet werden können. Diese Aufgabe wurde durch die Bereitstellung der Verbindungen der Formel I gelöst.

Es wurde gefunden, daß die Verbindungen der Formel I und ihre physiologisch unbedenklichen Säureadditionssalze wertvolle pharmakologische Eigenschaften besitzen. So zeigen sie insbesondere Wirkungen auf das Zentralnervensystem, vor allem dopaminstimulierende (Anti-Parkinson) Wirkungen. Im einzelnen induzieren die Verbindungen der Formel I contralaterales Drehverhalten in Hemiparkinson-Ratten (feststellbar nach der Methode von Ungerstedt et al., Brain Res. 24, (1970), 485-493) und hemmen die Bindung von tritiierten Dopaminagonisten und -antagonisten an striäire Rezeptoren (feststellbar nach den Methoden von Schwarcz et al., J. Neurochemistry, 34, (1980), 772-778, und Creese et al., European J. Pharmacol., 46, (1977), 377-381). Zusätzlich hemmen die Verbindungen den Zungen-Kieferreflex bei der narkotisierten Ratte (feststellbar in Anlehnung an die Methoden von Barnett et al., European J. Pharmacol. 21, (1973), 178-182, und von Ilhan et al., European J. Pharmacol. 33, (1975), 61-64). Weiterhin treten analgetische Wirkungen auf.

Verbindungen der Formel I und ihre physiologisch unbedenklichen Säureadditionssalze können daher als Arzneimittelwirkstoffe und auch als Zwischenprodukte zur Herstellung anderer Arzneimittelwirkstoffe verwendet werden.

Gegenstand der Erfindung sind die Indolderivate der Formel I sowie ihre physiologisch unbedenklichen Säureadditionssalze.

Im Rest Ind bedeutet Alkyl vorzugsweise Methyl, ferner auch Ethyl, n-Propyl, Isopropyl, n-Butyl, Isobutyl, sek.-Butyl oder tert.-Butyl. O-Alkyl ist vorzugsweise Methoxy, ferner auch Ethoxy, n-Propoxy, Isopropoxy, n-Butoxy, Isobutoxy, sek.-Butoxy oder tert.-Butoxy. S-Alkyl steht vorzugsweise für Methylthio, aber auch für Ethylthio, n-Propylthio, Isopropylthio, n-Butylthio, Isobutylthio, sek.-Butylthio oder tert.-Butylthio. SO-Alkyl steht vorzugsweise für Methylsulfinyl, ferner auch für Ethylsulfinyl, n-Propylsulfinyl, Isopropylsulfinyl, n-Butylsulfinyl, Isobutylsulfinyl, sek.-Butylsulfinyl oder tert.-Butylsulfinyl. $SO_2$-Alkyl ist vorzugsweise Methylsulfonyl, ferner auch Ethylsulfonyl, n-Propylsulfonyl, Isopropylsulfonyl, n-Butylsulfonyl, Isobutylsulfonyl, sek.-Butylsulfonyl oder tert.-Butylsulfonyl.

Der Rest Ind bedeutet insbesondere einen unsubstituierten Indol-3-yl-rest. Falls Ind jedoch einen substituierten Indol-3-yl-rest bedeutet, so ist er vorzugsweise einfach, insbesondere in der 2-, 5- oder 6-Stellung substituiert. Weiterhin ist eine Substitution in 1-, 4- oder 7-Stellung möglich. Bevorzugte disubstituierte Indol-3-yl-reste sind in 5,6-Stellung substituiert; Disubstitution ist auch in 1,2-, 1,4-, 1,5-, 1,6-, 1,7-, 2,4-, 2,5-, 2,6-, 2,7-, 4,5-, 4,6-, 4,7-, 5,7-oder 6,7-Stellung möglich. In allen diesen Fällen können die Substituenten gleich oder verschieden sein.

Im einzelnen sind die bevorzugten Substituenten im Benzolring des Restes Ind Methyl, Ethyl, Methoxy, Ethoxy, Methylthio, Ethylthio, OH, F, Cl, Br, $CF_3$ und CN. Einige bevorzugte Bedeutungen des Restes Ind sind dementsprechend Indol-3-yl, ferner 1-, 2-, 4-, 5-, 6- oder 7-Methylindol-3-yl, 1-, 2-, 4-, 5-, 6-oder 7-Ethylindol-3-yl, 4-, 5-, 6- oder 7-Methoxyindol-3-yl, 4-, 5-, 6- oder 7-Ethoxyindol-3-yl, 4-, 5-, 6- oder 7-Methylthioindol-3-yl, 4-, 5-, 6- oder 7-Ethylthioindol-3-yl, 4-, 5-, 6- oder 7-Methylsulfinylindol-3-yl. 4-, 5-, 6- oder 7-Methylsulfonyl-indol-3-yl, 4-, 5-, 6- oder 7-Hydroxyindol-3-yl, 4-, 5-, 6- oder 7-Fluorindol-3-yl-, 4-, 5-, 6- oder 7-Chlorindol-3-yl, 4-, 5-, 6- oder 7-Bromindol-3-yl, 4-, 5-, 6- oder 7-Trifluormethylindol-3-yl, 4-, 5-, 6-oder 7-Cyanindol-3-yl, 1,2-, 1,4-, 1,5-, 1,6-, 1,7-, 2,4-, 2,5-, 2,6-, 2,7-, 4,5-, 4,6-, 4,7-, 5,6-, 5,7-oder 6,7-Dimethylindol-3-yl, 1-Methyl-4-, -5-, -6- oder -7-methoxyindol-3-yl, 1-Methyl-4-, -5-, -6- oder -7-methylthioindol-3-yl, 1-Methyl-4-, -5-, -6-oder -7-fluorindol-3-yl, 1-Methyl-4-, -5-, -6- oder -7-chlorindol-3-yl, 1-Methyl-4-, -5-, -6- oder -7-bromindol-3-yl, 1-Methyl-4-, -5-, -6- oder -7-trifluormethylindol -3-yl, 1-Methyl-4-, -5-, -6- oder -7-cyanindol-3-yl, 2-Methyl-4-, -5-, -6- oder -7-methoxyindol-3-yl, 2-Methyl-4-, -5-, -6- oder 7-methylthioindol-3-yl, 2-Methyl-4-, -5-, -6- oder -7-fluorindol-3-yl, 2-Methyl-4-, -5-, -6- oder -7-chlorindol-3-yl, 2-Methyl-4-, -5-, -6- oder -7-bromindol-3-yl, 2-Methyl-4-, -5-, -6- oder -7-trifluormethylindol-3-yl, 2-Methyl-4-, -5-, -6- oder -7-cyanindol-3-yl, 4-Methyl-5-fluorindol-3-yl, 5-Fluor-6- oder -7-methylindol -3-yl, 4-Methyl-5-chlorindol-3-yl, 4-Chlor-5-methylindol-3-yl, 5-Methyl-6- oder 7-chlorindol-3-yl, 5-Chlor-6- oder -7-methylindol-3-yl, 4,5-, 4,6-, 4,7-, 5,6-, 5,7- oder 6,7-Dimethoxyindol-3-yl, 4,5-, 4,6-, 4,7-, 5,6-, 5,7-oder 6,7-Dichlorindol-3-yl, 4-Trifluormethyl-5-, -6-oder -7-chlorindol-3-yl.

Der Parameter n ist vorzugsweise 4. Die Gruppe A ist also vorzugsweise $-(CH_2)_4-$.

Der Rest Ar ist bevorzugt m-Hydroxyphenyl, ferner o- oder p-Hydroxyphenyl.

Dementsprechend sind Gegenstand der Erfindung insbesondere diejenigen Verbindungen der Formel I, in denen mindestens einer der genannten Reste eine der vorstehend angegebenen, insbesondere der vorstehend angegebenen bevorzugten Bedeutungen hat. Einige bevorzugte Gruppen von Verbindungen können durch die folgenden Teilformeln Ia bis In ausgedrückt werden, die der Formel I entsprechen und worin die nicht näher bezeichneten Reste und Parameter die bei Formel I angegebene Bedeutung haben, worin jedoch

- in Ia Ind - Indol-3-yl, Methylindol-3-yl, Dimethylindol -3-yl, Methoxyindol-3-yl, Dimethoxyindol -3-yl, Hydroxyindol-3-yl, Dihydroxyindiol -3-yl, Fluorindol-3-yl, Chlorindol-3-yl, Dichlorindol-3-yl, Bromindol-3-yl, Cyanindol-3-yl oder Methylendioxyindol-3-yl bedeutet, wobei die Substituenten vorzugsweise in 5- und/oder 6-Stellung stehen;
- in Ib Ind - Indol-3-yl, 5- oder 6-Methylindol-3-yl, 5,6-Dimethylindol-3-yl, 5- oder 6-Methoxyindol -3-yl, 5,6-Dimethoxyindol-3-yl oder 5-Cyanindol-3-yl bedeutet;
- in Ic Ind - Indol-3-yl bedeutet;
- in Id A - $-(CH_2)_4-$ bedeutet;
- in Ie Ind - Indol-3-yl, A - $-(CH_2)_4-$ und Ar - 3- oder 4-Hydroxyphenyl bedeuten;
- in If Ind - Indol-3-yl, A - $-(CH_2)_4-$ und Ar - 3-Hydroxyphenyl bedeuten;
- in Ig Ar - 4-Hydroxyphenyl bedeutet;
- in Ih die beiden Gruppen L zusammen eine C-C-Bindung bedeuten.

Die Verbindungen der Formel I können ein oder mehrere asymmetrische Kohlenstoffatome besitzen. Sie können daher als Racemate, falls mehrere asymmetrische Kohlenstoffatome vorhanden sind, auch als Gemische mehrerer Racemate sowie in verschiedenen optischaktiven Formen vorliegen.

Gegenstand der Erfindung ist ferner ein Verfahren zur Herstellung der Verbindungen der Formel I sowie ihrer physiologisch unbedenklichen Säureadditionssalze, dadurch gekennzeichnet, daß man eine Verbindung der allgemeinen Formel II

$$Ind-A-X^1 \quad II$$

worin

$X^1$ X oder $NH_2$ und

X Cl, Br, J, OH oder eine reaktionsfähig funktionell abgewandelte OH-Gruppe bedeuten und

Ind und A die angegebenen Bedeutungen haben, mit einer Verbindung der allgemeinen Formel III

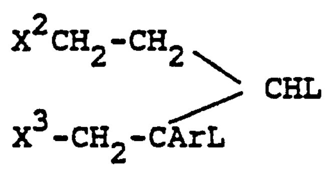

III

worin

$X^2$ und $X^3$ gleich oder verschieden sein können und, falls $X^1 = NH_2$ ist, jeweils X, andernfalls zusammen NH bedeuten und

Ar und L die angegebenen Bedeutungen haben umsetzt

oder daß man eine sonst der Formel I entsprechende Verbindung, die jedoch an Stelle eines oder mehrerer Wasserstoffatome eine oder mehrere reduzierbare Gruppe(n) und/oder eine oder mehrere zusätzliche C-C- und/oder C-N-Bindung(en) enthält, mit einem reduzierenden Mittel behandelt oder daß man eine sonst der Formel I entsprechende Verbindung, die jedoch an Stelle eines oder mehrerer Wasserstoffatome eine oder mehrere solvolytisch abspaltbare Gruppe(n) enthält, mit einen solvolysierenden Mittel behandelt

oder daß man zur Herstellung einer Verbindung der Formel I, worin die beiden Reste L zusammen eine C-C-Bindung bedeuten, eine Verbindung der Formel IV

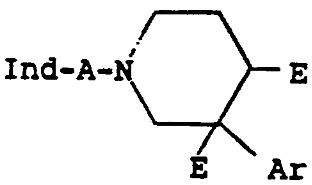

IV

worin
der eine Rest E X, CN oder $NH_2$,
der andere Rest E H bedeutet und
Ind, A, Ar und X die angegebenen Bedeutungen haben mit einem HE-abspaltenden Mittel behandelt

und/oder daß man gegebenenfalls in einer Verbindung der Formel I eine Thioethergruppe zu einer SO-Gruppe oder $SO_2$-Gruppe oder eine SO-Gruppe zu einer $SO_2$-Gruppe oxydiert und/oder eine Alkoxygruppe unter Bildung einer OH-Gruppe spaltet und/oder daß man eine erhaltene Base der Formel I durch Behandeln mit einer Säure in eines ihrer physiologisch unbedenklichen Säureadditionssalze umwandelt.

Die Herstellung der Verbindungen der Formel I erfolgt im übrigen nach an sich bekannten Methoden, wie sie in der Literatur (z. B. in den Standardwerken wie Houben-Weyl, Methoden der Organischen Chemie, Georg-Thieme-Verlag, Stuttgart; Organic Reactions, John Wiley & Sons, Inc., Hew York) beschrieben sind, und zwar unter Reaktionsbedingungen, wie sie für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

In den Indolderivaten der Formel II ist $X^1$ vorzugsweise X; dementsprechend sind in den Verbindungen der Formel III $X^2$ und $X^3$ vorzugsweise zusammen NH. Der Rest X ist vorzugsweise Cl oder Br; er kann jedoch auch J, OH oder eine reaktionsfähig funktionell abgewandelte OH-Gruppe bedeuten, insbesondere Alkylsulfonyloxy mit 1 - 6 (z. B. Methansulfonyloxy) oder Arylsulfonyloxy mit 6 - 10 C-Atomen (z. B. Benzolsulfonyloxy, p-Toluolsulfonyloxy, 1- oder 2-Naphthalinsulfonyloxy)

Dementsprechend sind die Indolderivate der Formel I insbesondere durch Umsetzung der Verbindungen der Formeln Ind-A-Cl oder Ind-A-Br mit Verbindungen der Formel III, worin $X^2$ und $X^3$ zusammen eine NH-Gruppe bedeuten (nachstehend als IIIa bezeichnet) erhältlich.

Die Verbindungen der Formeln II und III sind zum Teil bekannt; die nicht bekannten Verbindungen der Formeln II und III können leicht analog zu den bekannten Verbindungen hergestellt werden. So sind einige der Verbindungen der Formel II aus der DE-OS-2 827 874 bekannt.

Alkohole der Formel Ind-A-OH sind z. B. durch Reduktion der entsprechenden Carbonsäuren oder ihrer Ester erhältlich. Behandeln mit Tionylchlorid, Bromwasserstoff, Phosphortribromid oder ähnlichen Halogenverbindungen liefert die entsprechenden Halogenide der Formel Ind-A-Hal (worin Hal Cl, Br oder J bedeutet). Die entsprechenden Sulfonyloxyverbindungen sind erhältlich aus den Alkoholen Ind-A-OH durch Umsetzung mit den entsprechenden Sulfonsäurechloriden. Die Jodverbindungen der Formel Ind-A-J, z. B. 3-(4-Jodbutyl)-indol, sind z. B. durch Einwirkung von Kaliumjodid auf die zugehörigen p-Toluolsulfonsäureester erhältlich. Die Amine der Formel Ind-A-$NH_2$ sind z. B. aus den Halogeniden mit Phthalimidkalium oder durch Reduktion der entsprechenden Nitrile zugänglich.

Die Piperidinderivate IIIa sind z. B. erhältlich durch Umsetzung von 3-Piperidon mit metallorganischen Verbindungen der Formel M-Ar (worin M ein Li-Atom oder MgHal bedeutet), anschließende Hydrolyse zu den entsprechenden 3-Ar-3-hydroxy-piperidinen sowie gewünschtenfalls nachfolgende Dehydratisierung zu 3-Ar-3,4-dehydro-piperidinen. Verbindungen der Formel III ($X^2$ und $X^3$ jeweils X) sind z. B. herstellbar durch Reduktion von 2-Ar-glutarsäureestern zu 2-Ar-1,5-pentandiolen and gegebenenfalls anschließende Umsetzung mit $SOCl_2$ bzw. $PBr_3$.

Die Umsetzung der Verbindungen II und III verläuft nach Methoden, wie sie für die Alkylierung von Aminen aus der Literatur bekannt sind. Man kann ohne Gegenwart eines Lösungsmittels die Komponenten miteinander verschmelzen, gegebenenfalls im geschlossen Rohr oder im Autoklaven. Es ist aber auch möglich, die Verbindungen in Gegenwart eines indifferenten Lösungsmittels umzusetzen. Als Lösungsmittel eignen sich z. B. Kohlenlwasserstoffe, wie Benzol, Toluol, Xylol; Ketone wie Aceton, Butanon; Alkohole wie Methanol, Ethanol, Isopropanol, n-Butanol; Ether wie Tetrahydrofuran (THF) oder Dioxan; Amide wie Dimethylformamid (DMF) oder N-Methylpyrrolidon; Nitrile wie Acetonitril, gegebenenfalls auch Gemische dieser Lösungsmittel untereinander oder Gemische mit Wasser. Der Zusatz eines säurebindenden Mittels, beispielsweise eines Alkali- oder Erdalkalimetallhydroxids, -carbonats oder -bicarbonats oder eines anderen Salzes einer schwachen Säure der Alkali- oder Erdalkalimetalle, vorzugsweise des Kaliums, Natriums oder Calciums, oder der Zusatz einer organischen Base wie Triethylamin, Dimethylanilin, Pyridin oder Chinolin oder eines Überschusses der Aminkomponente Ind-A-$NH_2$ bzw. des Piperidinderivates der Formel IIIa kann günstig sein. Die Reaktionszeit liegt je nach den angewendeten Bedingungen zwischen einigen Minuten und 14 Tagen, die Reaktionstemperatur zwischen etwa 0 und 150°, normalerweise zwischen 20 und 130°.

Es ist ferner möglich, eine Verbindung der

Formel I zu erhalten, indem man ein Vorprodukt, das an Stelle von Wasserstoffatomen eine oder mehrere reduzierbare Gruppe(n) und/oder eine oder mehrere zusätzliche C-C- und/oder C-N-Bindung(en) enthält, mit reduzierenden Mitteln behandelt, vorzugsweise bei Temperaturen zwischen -80 und +250° in Gegenwart mindestens eines inerten Lösungsmittels.

Reduzierbare (durch Wasserstoff ersetzbare) Gruppen sind insbesondere Sauerstoff in einer Carbonylgruppe, Hydroxyl, Arylsulfonyloxy (z. B. p-Toluolsulfonyloxy), N-Benzolsulfonyl, N-Benzyl oder O-Benzyl.

Es ist grundsätzlich möglich, Verbindungen, die nur eine, oder solche, die nebeneinander zwei oder mehr der oben angeführten Gruppen bzw. zusätzlichen Bindungen enthalten, reduktiv in eine Verbindung der Formel I überzuführen. Vorzugsweise bedient man sich hierzu des nascierenden Wasserstoffs oder komplexer Metallhydride, ferner der Reduktion nach Wolff-Kishner.

Bevorzugte Ausgangsstoffe für die Reduktion entsprechen der Formel V

Ind'-G-Q'-Ar' V

worin

Ind' einen Indol-3-ylrest, der ein- oder zweifach durch Alkyl, O-Alkyl, S-Alkyl, SO-Alkyl, $SO_2$-Alkyl, OH, F, Cl, Br, $CF_3$, CN und/oder O-Benzyl oder durch eine Methylendioxygruppe und/oder durch eine Arylsulfonylgruppe oder eine Benzylgruppe in 1-Stellung substituiert sein kann,

G eine $-(CH_2)_n$-Kette, worin jedoch eine oder mehrere $-CH_2$-Gruppe(n) durch -CO- und/oder ein oder mehrere Wasserstoffatome durch OH-Gruppen ersetzt sein können,

Q' Q oder 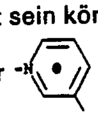 An⊖

An⊖ ein Anion einer starken Säure und

Ar' eine einfach durch OH oder O-Benzyl substituierte Phenylgruppe bedeuten,

worin jedoch nicht gleichzeitig Ind' = Ind, G = A, Q' = Q und Ar' = Ar sein können.

In den Verbindungen der Formel V ist G bevorzugt $-CH_2CH_2CO-$, $-(CH_2)_3-CO-$ oder $-(CH_2)_4-CO-$.

Verbindungen der Formel V sind z. B. herstellbar durch Umsetzung von 3-Ar'-1, 2, 3, 6-tetrahydropyridinen, 3-Ar'-piperidinen oder 3-Ar'-pyridinen mit Verbindungen der Formel VI

Ind'-G-X[1] VI

worin

Ar', Ind', G und X[1] die oben angegebenen Bedeutungen haben,

unter den Bedingungen, die oben für die Umsetzung von II mit III angegeben sind.

Säureamide der Formel V [G = $-(CH_2)_{n-1}-CO-$, Q' = Q] sind z. B. aus den freien Carbonsäuren der Formel Ind'-$(CH_2)_{n-1}$-COOH und Aminen der Formel IIIa erhältlich, zweckmäßig in Gegegenwart eines Dehydratisierungsmittels, z. B. Carbonyldiimidazol oder Dicyclohexylcarbodiimid, in einem inerten Lösungsmittel, bevorzugt THF.

Wird als Reduktionsmittel nascierender Wasserstoff verwendet, so kann man diesen z. B. durch Behandlung von Metallen mit schwachen Säuren oder mit Basen erzeugen. So kann man z. B. ein Gemisch von Zink mit Alkalilauge oder von Eisen mit Essigsäure verwenden. Geeignet ist auch die Verwendung von Natrium oder einem anderen Alkalimetall in einem Alkohol wie Ethanol, Isopropanol, Butanol, Amyl- oder Isoamylalkohol oder Phenol. Man kann ferner eine Aluminium-Nickel-Legierung in alkalischwässeriger Lösung, gegebenenfalls unter Zusatz von Ethanol, verwenden. Auch Natrium- oder Aluminiumamalgam in wässerigalkoholischer oder wässeriger Lösung sind zur Erzeugung des nascierenden Wasserstoffs geeignet. Die Umsetzung kann auch in heterogener Phase durchgeführt werden, wobei man zweckmäßig eine wässerige und eine Benzol- oder Toluol-Phase verwendet.

Als Reduktionsmittel können ferner besonders vorteilhaft komplexe Metallhydride, wie $LiAlH_4$, $NaBH_4$, Diisobutylaluminiumhydrid oder $NaAl(OCH_2CH_2OCH_3)_2H_2$ sowie Diboran eingesetzt werden, falls erwünscht unter Zusatz von Katalysatoren wie $BF_3$, $AlCl_3$ oder LiBr.

Als Lösungsmittel eignen sich hierfür insbesondere Ether wie Diethylether, Di-n-butylether, THF, Dioxan, Diglyme oder 1,2-Dimethoxyethan sowie Kohlenwasserstoffe wie Benzol. Für eine Reduktion mit $NaBH_4$ sind in erster Linie Alkohole wie Methanol oder Ethanol, ferner Wasser sowie wässerige Alkohole als Lösungsmittel geeignet. Nach diesen Methoden reduziert man vorzugsweise bei Temperaturen zwischen -80 und +150°, insbesondere zwischen etwa 0 und etwa 100°.

Besonders vorteilhaft lassen sich -CO-Gruppen in Säureamiden (z. B. solchen der Formel V, worin G = $-(CH_2)_{n-1}-CO-$ und Q' = Q ist) mit $LiAlH_4$ in THF bei Temperaturen zwischen etwa 0 und 66° zu $CH_2$-Gruppen reduzieren. Dabei können in 1-Stellung des Indolrings befindliche Arylsulfonyl-Schutzgruppen gleichzeitig reduktiv abgespalten werden.

Eine Reduktion der Pyridiniumsalze der Formel V (worin Q' 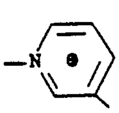 An⊖ und An vorzugsweise Cl oder Br bedeutet) zu Verbindungen der Formel I gelingt z. B. mit $NaBH_4$ in Wasser, Methanol, Ethanol oder in Gemischen dieser Lösungsmittel, falls erwünscht unter Zusatz einer Base wie NaOH, bei Temperaturen zwischen etwa 0 und 80°.

N-Benzylgruppen können reduktiv mit Natrium in flüssigem Ammoniak abgespalten werden.

Es ist ferner möglich, eine oder mehrere Carbonylgruppen nach der Methode von Wolff-Kishner zu $CH_2$ Gruppen zu reduzieren, z. B. durch Behandlung mit wasserfreiem Hydrazin in absolutem Ethanol unter Druck bei Temperaturen zwischen etwa 150 und 250°.

Als Katalysator wird vorteilhaft Natriumalkoholat verwendet. Die Reduktion kann auch nach der Methode von Huang-Minlon variiert werden, indem man mit Hydrazinhydrat in einem hochsiedenden, mit Wasser mischbaren Lösungsmittel, wie Diethylenglykol oder Triethylenglykol, in Gegenwart von Alkali, wie Natriumhydroxid, umsetzt. Das Reaktionsgemisch wird in der Regel etwa 3 - 4 Stunden gekocht. Anschließend wird das Wasser abdestilliert und das gebildete Hydrazon bei Temperaturen bis zu etwa 200° zersetzt. Die Wolff-Kishner-Reduktion kann auch bei Raumtemperatur in Dimethylsulfoxid mit Hydrazin ausgeführt werden.

Verbindungen, die sonst der Formel I entsprechen, aber an Stelle eines oder mehrerer H-Atome eine oder mehrere solvolytisch abspaltbare Gruppe(n) enthalten, können zu den Verbindungen der Formel I solvolysiert, insbesondere hydrolysiert werden. Die Ausgangsstoffe für die Solvolyse sind beispielsweise erhältlich durch Reaktion von IIIa mit Verbindungen, die der Formel II ($X^1 = X$) entsprechen, aber an Stelle eines oder mehrerer H-Atome eine oder mehrere solvolytisch abspaltbare Gruppe(n) enthalten. So können 1-Acylindolderivate (entsprechend der Formel I, aber in 1-Stellung des Ind-Restes eine Acylgruppe enthaltend, vorzugsweise eine Alkanoyl-, Alkylsulfonyl- oder Arylsulfonylgruppe mit jeweils bis zu 10 C-Atomen, wie Methan-, Benzol- oder p-Toluolsulfonyl) zu den entsprechenden in der 1-Stellung des Indolringes unsubstituierten Indolderivaten hydrolysiert werden, z. B. in saurem, besser in neutralem oder alkalischem Medium bei Temperaturen zwischen 0 und 200°. Als basische Katalysatoren verwendet man zweckmäßig Natrium-, Kalium- oder Calciumhydroxid, Natrium- oder Kaliumcarbonat, oder Ammoniak. Als Lösungsmittel wählt man vorzugsweise Wasser, niedere Alkohole wie Methanol, Ethanol; Ether wie THF, Dioxan; Sulfone wie Tetramethylensulfon; oder deren Gemische. Eine Hydrolyse kann auch bereits beim Behandeln mit Wasser allein erfolgen, insbesondere in der Siedehitze.

Man gelangt ferner zu Verbindungen der Formel I, indem man aus Verbindungen der Formel IV unter Ausbildung einer Doppelbindung HE abspaltet. Entsprechend der Definition von E kann es sich z. B. handeln um eine Abspaltung von Halogenwasserstoff, Wasser (Dehydratisierung), einer Carbonsäure oder einer anderen Säure, von Ammoniak oder von HCN. Die Ausgangsstoffe der Formel IV sind z. B. erhältlich durch Umsetzung von II ($X^1 = X$) mit einer Verbindung der Formel VII

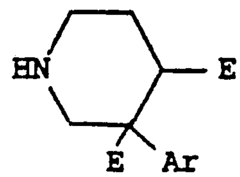

VII

worin E und Ar die angegebenen Bedeutungen haben.

Falls einer der Reste E = Hal ist, kann dieser Substituent unter basischen Reaktionsbedingungen leicht eliminiert werden. Als Basen können verwendet werden: Alkalimetallhydroxide, Alkalimetallcarbonate, Alkoholate, wie z. B. Kalium-tert.-butylat, Amine, wie z. B. Dimethylanilin, Pyridin, Collidin oder Chinolin; als Lösungmittel benutzt man z. B. Benzol, Toluol, Cyclohexan, Methanol, Dioxan, THF oder tert.-Butanol. Die als Basen verwendeten Amine können auch im Überschuß als Lösungsmittel eingesetzt werden. Bedeutet der eine der Reste E eine OH-Gruppe, so benutzt man als wasserabspaltende Mittel vorzugsweise Säuren wie Essigsäure, Salzsäure oder Gemische beider. Der Zusatz eines Lösungsmittels (z. B. Wasser oder Ethanol) kann von Vorteil sein. Die Eliminierung von Acyl-, Alkylsulfonyl sowie Alkoxysulfonyloxy- oder Aminoresten kann unter ähnlichen Bedingungen durchgeführt werden. Eine Eliminierung von Sulfonsäureresten, z. B. die der Mesylate oder Tosylate, erfolgt schonend durch Kochen in DMF oder Dimethylsulfoxid mit Alkalimetallcarbonaten z. B. $Li_2CO_3$ oder mit Kaliumacetat. Ammoniak kann bereits durch Erhitzen der Salze der entsprechenden Aminoverbindungen (insbesondere der 3-Aminoderivate) abgespalten werden. In ähnlicher Weise kann HCN aus Verbindungen der Formel IV (eine Gruppe E = CN) durch Erhitzen abgespalten werden. Die Eliminierung von HE aus IV erfolgt allgemein bei Temperaturen zwischen etwa 0 und etwa 250°, vorzugsweise zwischen 50 und 200°.

Weiterhin kann man in einem Thioether der Formel I die Thioethergruppe zu einer SO-Gruppe oder zu einer $SO_2$-Gruppe oder in einem Sulfoxid der Formel I die SO-Gruppe zu einer $SO_2$-Gruppe oxydieren. Die zu oxydierenden Thioether- oder Sulfoxidgruppen können als Substituenten im Rest Ind vorhanden sein. Will man die Sulfoxide erhalten, so oxydiert man beispielsweise mit Wasserstoffperoxid, Persäuren wie m-Chlorperbenzoesäure, Cr(VI)-Verbindungen wie Chromsäure, $KMnO_4$, l-Chlorbenztriazol, Ce(IV)-Verbindungen wie $(NH_4)_2Ce(NO_3)_6$, negativ substituierten aromatischen Diazoniumsalzen wie o- oder p-Nitrophenyldiazoniumchlorid oder elektrolytisch unter verhältnismäßig milden Bedingungen und bei relativ niedrigen Temperaturen (etwa -80 bis +100°). Will man dagegen die Sulfone (aus den Thioethern oder den Sulfoxiden) erhalten, so verwendet man die gleichen Oxydationsmittel unter kräftigeren Bedingungen und/oder im Überschuß sowie in

der Regel bei höheren Temperaturen. Bei diesen Umsetzungen können die üblichen inerten Lösungsmittel zugegen oder abwesend sein. Als inerte Lösungsmittel eignen sich beispielsweise Wasser, wässerige Mineralsäuren, wässerige Alkalilaugen, niedere Alkohol wie Methanol oder Ethanol, Ester wie Ethylacetat, Ketone wie Aceton, niedere Carbonsäuren wie Essigsäure, Nitrile wie Acetonitril, Kohlenwasserstoffe wie Benzol, chlorierte Kohlenwasserstoffe wie Chloroform oder $CCl_4$. Ein bevorzugtes Oxydationsmittel ist 30 %-iges wässeriges Wasserstoffperoxid. Dieses führt bei Anwendung der berechneten Menge in Lösungsmitteln wie Essigsäure, Aceton, Methanol, Ethanol oder wässeriger Natronlauge bei Temperaturen zwischen -20 und 100° zu den Sulfoxiden, im Überschuß bei höheren Temperaturen, vorzugsweise in Essigsäure oder in einem Gemisch aus Essigsäure und Acetanhydrid, zu den Sulfonen.

Ether der Formel I, in denen der Rest Ind ein- oder zweifach durch O-Alkyl substituiert sind, können nach Methoden, die aus der Literatur bekannt sind, gespalten werden, wobei die entsprechenden Hydroxyderivate entstehen. z. B. kann man die Ether spalten durch Behandeln mit HBr oder HJ in wässeriger oder essigsaurer Lösung, durch Erhitzen mit Lewis-Säuren wie $AlCl_3$ oder Bortrihalogeniden oder durch Verschmelzen mit Pyridin- oder Anilinhydrohalogeniden, vorzugsweise Pyridinhydrochlorid, bei etwa 150 - 250°. Besonders schonend ist die reduktive Spaltung mit Diisobutylaluminiumhydrid (Methode vgl. Synthesis 1975, 617).

Eine erhaltene Base der Formel I kann mit einer Säure in das zugehörige Säureadditionssalz übergeführt werden. Für diese Umsetzung eignen sich Säuren die physiologisch unbedenkliche Salze liefern. So können anorganische Säuren verwendet werden, z. B. Schwefelsäure, Halogenwasserstoffsäuren wie Chlorwasserstoffsäure oder Bromwasserstoffsäure, Phosphorsäuren wie Orthophosphorsäure, Salpetersäure, Sulfaminsäure, ferner organische Säuren, im einzelnen aliphatische, alicyclische, araliphatische, aromatische oder heterocyclische ein- oder mehrbasige Carbon-, Sulfon- oder Schwefelsäuren, wie Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Apfelsäure, Benzoesäure, Salicylsäure, 2-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono und -disulfonsäuren, Laurylschwefelsäure.

Die freien Basen der Formel I können, falls gewünscht, aus ihren Salzen durch Behandlung mit starken Basen wie Natrium- oder Kaliumhydroxid, Natrium oder Kaliumcarbonat in Freiheit gesetzt werden.

Gegenstand der Erfindung ist ferner die Verwendung der Verbindungen der Formel I und ihrer physiologisch unbedenklichen Salze zur Herstellung pharmazeutischer Zubereitungen, insbesondere auf nicht-chemischem Wege. Hierbei können sie zusammen mit mindestens einem Träger- oder Hilfsstoff und gegebenenfalls in Kombination mit einem oder mehreren weiteren Wirkstoff(en) in eine geeignete Dosierungsform gebracht werden.

Gegenstand der Erfindung sind ferner Mittel, insbesondere pharmazeutische Zubereitungen, enthaltend mindestens eine Verbindung der Formel I und/oder eines ihrer physiologisch unbedenklichen Salze. Diese Zubereitungen können als Arzneimittel in der Human- oder Veterinärmedizin eingesetzt werden. Als Trägersubstanzen kommen organische oder anorganische Stoffe in Frage, die sich für die enterale (z. B. orale), parenterale oder topische Applikation eignen und mit den neuen Verbindungen nicht reagieren, beispielsweise Wasser, pflanzliche Öle, Benzylalkohole, Polyethylenglykole, Gelatine, Kohlehydrate wie Lacto se oder Stärke, Magnesiumstearat, Talk, Vaseline®. Zur enteralen Applikation dienen insbesondere Tabletten, Dragees, Kapseln, Sirupe, Säfte, Tropfen oder Suppositorien, zur parenteralen Applikation Lösungen, vorzugsweise ölige oder wässerige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z. B. zur Herstellung von Injektionspräparaten verwendet werden.

Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z. B. ein oder mehrere Vitamine.

Die Verbindungen der Formel I und ihre physiologisch unbedenklichen Salze können verwendet werden bei der therapeutischen Behandlung des menschlichen oder tierischen Körpers und bei der Bekämpfung von Krankheiten, insbesondere von Parkinsonismus, von extrapyramidalen Störungen bei der Neuroleptikatherapie, von Depressionen und/oder Psychosen und von Nebenwirkungen bei der Behandlung der Hypertonie (z. B. mit α-Methyldopa). Ferner können die Verbindungen in der Endokrinologie und Gynäkologie Verwendung finden, z. B. zur Therapie von Akromegalie, Hypogonadismus, sekundärer Amenorrhoe, prämenstruellem Syndrom, unerwünschter puerperaler Laktation und generell als Prolaktin-Hemmer, weiterhin zur Therapie cerebraler Störungen (z. B. Migräne),

insbesondere in der Geriatrie ähnlich wie gewisse Ergot-Alkaloide

Dabei werden die erfindungsgemäßen Substanzen in der Regel in Analogie zu bekannten, im Handel befindlichen Präparaten (z. B. Bromocriptin; Dihydroergocornin) verabreicht. vorzugsweise in Dosierungen zwischen etwa 0,2 und 500 mg, insbesondere zwischen 0,2 und 50 mg pro Dosierungseinheit. Die tägliche Dosierung liegt vorzugsweise zwischen etwa 0,001 und 10 mg/kg Körpergewicht. Die niedrigen Dosierungen (etwa 0,2 bis 1 mg pro Dosierungseinheit; etwa 0,001 bis 0,005 mg/kg Körpergewicht) kommen dabei insbesondere für die Verwendung als Migränemittel in Betracht; für die übrigen Indikationen werden Dosierungen zwischen 10 und 50 mg pro Dosierungseinheit bevorzugt. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

In den nachfolgenden Beispielen bedeutet "übliche Aufarbeitung":

Man gibt, falls erforderlich, Wasser hinzu, extrahiert mit einem organischen Lösungsmittel wie Toluol, Chloroform oder Dichlormethan, trennt ab, trocknet die organische Phase über Natriumsulfat, filtriert, dampft ein und reinigt durch Chromatographie und/oder Kristallisation. Temperaturen sind in Celsiusgraden angegeben, Rf-Werte an Kieselgel (Toluol/Triethylamin 8 : 2, wenn nicht anders angegeben).

**Beispiel 1**

Man rührt eine Lösung von 2,08 g 3-(4-Chlorbutyl)-indol [oder 2,52 g 3-(4-Brombutyl)-indol] und 1,6 g 3-Phenyl-1,2,3,6-tetrahydropyridin in 10 ml Acetonitril 12 Stunden bei 20°, arbeitet wie üblich auf und erhält 3-[4-(3-Phenyl-1,2,3,6-tetrahydropyridyl)butyl]-indol. Hydrochlorid, F. 209 - 212°. (Dieses Verfahrensprodukt ist keine Verbindung der Formel I).

Analog erhält man aus den entsprechenden Chlor- oder Bromalkyl-indolen mit den entsprechenden 3-Aryl-1,2,3,6-tetrahydropyridinen bzw. -piperidinen:

3-[3-(3-m-Hydroxyphenyl-1,2,3,6-tetrahydropyridyl)-propyl]-indol

3[3-(3-p-Hydroxyphenyl-1,2,3,6-tetrahydropyridyl)-propyl]-indol

3-[4-(3-m-Hydroxyphenyl-1,2,3,6-tetrahydropyridyl)-butyl]-indol, Rf 0,32 ($CH_2Cl_2$/Methanol 9 : 1)

3-[4-(3-p-Hydroxyphenyl-1,2,3,6-tetrahydropyridyl)butyl]-indol

3-[4-(3-m-Hydroxyphenyl-1,2,3,6-tetrahydropyridyl)-butyl]-5-hydroxyindol

3-[4-(3-m-Hydroxyphenyl-1,2,3,6-tetrahydropyridyl)-butyl]-6-hydroxyindol

3-[5-(3-m-Hydroxyphenyl-1,2,3,6-tetrahydropyridyl)-pentyl]-indol

3-[5-(3-p-Hydroxyphenyl-1,2,3,6-tetrahydropyridyl)-pentyl]-indol

3-[3-(3-m-Hydroxyphenyl-1-piperidyl)-propyl]-indol

3-[3-(3-p-Hydroxyphenyl-1-piperidyl)-propyl]-indol

3-[4-(3-o-Hydroxyphenyl-1-piperidyl)-butyl]-indol

3-[4-(3-m-Hydroxyphenyl-1-piperidyl)-butyl]-indol, F. 135 - 143° (Zersetzung)

3-[4-(3-p-Hydroxyphenyl-1-piperidyl)-butyl]-indol

3-[4-(3-m-Hydroxyphenyl-1-piperidyl)-butyl]-5-hydroxyindol, F. 102 - 104°

3-[4-(3-m-Hydroxyphenyl-1-piperidyl)-butyl]-6-hydroxyindol, Rf 0,76 ($CH_2Cl_2$/$CH_3OH$/Triethylamin 7 : 2 : 1)

3-[4-(3-p-Hydroxyphenyl-1-piperidyl)-butyl]-5-hydroxyindol, F. 182 - 185°

3-[5-(3-m-Hydroxyphenyl-1-piperidyl)-pentyl]-indol

3-[5-(3-p-Hydroxyphenyl-1-piperidyl)-pentyl]-indol

3-[4-(3-m-Hydroxyphenyl-1-piperidyl)-butyl]-4-hydroxy-7-chlor-indol

3-[4-(3-p-Hydroxyphenyl-1-piperidyl)-butyl]-4-hydroxy-7-chlor-indol.

**Beispiel 2**

Zu einer Lösung von 4,23 g 1-[4-(3-Indolyl)-butyl]-3-m-hydroxyphenylpyridinium-bromid [erhältlich aus 3-(4-Brombutyl)-indol und 3-m-Hydroxyphenyl-pyridin] in 50 ml In NaOH gibt man unter Rühren 1 g $NaBH_4$ in 20 ml Wasser und rührt danach noch 3 Stunden bei 60°. Nach üblicher Aufarbeitung erhält man 3-[4-(3 m-Hydroxyphenyl-1,2,3,6-tetrahydropyridyl)-butyl]-indol, Rf 0,32 ($CH_2Cl_2$/Methanol 9 : 1)

Analog erhält man durch Reduktion der entsprechenden Pyridiniumbromide die anderen in Beispiel 1 angegebenen Tetrahydropyridine der Formel I.

**Beispiel 3**

Man kocht 4,88 g 1-Benzolsulfonyl-3-[4-3-m-hydroxyphenyl -1-piperidyl)-butyl]-indol (erhältlich aus 1-Benzolsulfonyl-3-(4-chlorbutyl)-indol und 3-m-Hydroxyphenylpiperidin) mit 1 g KOH in 7 ml Wasser und 14 ml Ethanol 16 Stunden, konzentriert das Gemisch, arbeitet wie üblich auf und erhält 3-[4-(3-m-Hydroxyphenyl -1-piperidyl)-butyl]-indol, F. 135 - 143° (Zersetzung).

**Beispiel 4**

Zu einer Lösung von 3,62 g 3-[4-(3-m-Methoxyphenyl-1-piperidyl)-butyl]-indol in 50 ml 1,2-Dichlorethan tropft man unter Rühren eine Lösung von 12,3 g Bortribromid-Dimethylsulfid-Komplex in 50 ml 1,2-Dichlorethan bei 60 - 80°. Nach Abkühlen und üblicher Aufarbeitung erhält man 3-[4-(3-m-Hydroxyphenyl-1-piperidyl)-butyl]-indol, F. 135 - 143° (Zersetzung).

Die nachstehenden Beispiele betreffen pharmazeutische Zubereitungen, die Amine der Formel I oder ihre Säureadditionssalze enthalten:

**Beispiel A:** Tabletten

Ein Gemisch von 1 kg 3-[4-(3-m-Hydroxyphenyl-1-piperidyl)-butyl]-indol, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

**Beispiel B** Dragees

Analog Beispiel A werden Tabletten gepreßt, die anschließend in üblicher Weise mit einem Überzug aus Saccharose, Kartoffelstärke, Talk, Tragant und Farbstoff überzogen werden.

**Beispiel C** Kapseln

2 kg 3-[4-(3-m-Hydroxyphenyl-1-piperidyl)-butyl]-indol werden in üblicher Weise in Hartgelatinekapseln gefüllt, so daß jede Kapsel 20 mg des Wirkstoffs enthält.

**Beispiel D** Ampullen

Eine Lösung von 1 kg 3-[4-(3-m-Hydroxyphenyl-1-piperidyl)-butyl]-indol-hydrochlorid in 30 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

Analog sind Tabletten, Dragees, Kapseln und Ampullen erhältlich, die einen oder mehrere der übrigen Wirkstoffe der Formel 1 und/oder ihre physiologisch unbedenklichen Säureadditionssalze enthalten.

**Patentansprüche**

1. Indolderivate der allgemeinen Formel I

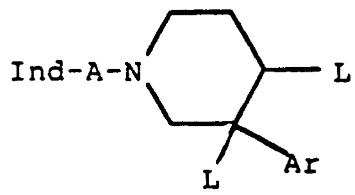

I

worin

Ind einen Indol-3-ylrest, der ein- oder zweifach durch Alkyl, O-Alkyl, S-Alkyl, SO-Alkyl, $SO_2$-Alkyl, OH, F, Cl, Br, $CF_3$ und/oder CN oder durch eine Methylendioxygruppe substituiert sein kann,
A $-(CH_2)_n-$,
n 3, 4 oder 5,
Ar eine Hydroxyphenylgruppe und
die beiden Reste L jeweils H oder zusammen eine C-C-Bindung

bedeuten und
worin die Alkylgruppen jeweils 1 - 4 C-Atome besitzen

sowie deren physiologisch unbedenkliche Säureadditionssalze.

2. 3-[4-(3-m-Hydroxyphenyl-1-piperidyl)-butyl]-indol.

3. Verfahren zur Herstellung von Indolderivaten der allgemeinen Formel I

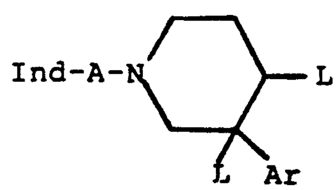

I

worin

Ind einen Indol-3-ylrest, der ein- oder zweifach durch Alkyl, O-Alkyl, S-Alkyl, SO-Alkyl, $SO_2$-Alkyl, OH, F, Cl, Br, $CF_3$ und/oder CN oder durch eine Methylendioxygruppe substituiert sein kann,
A $-(CH_2)_n-$,
n 3, 4 oder 5,
Ar eine Hydroxyphenylgruppe und
die beiden Reste L jeweils H oder zusammen eine Doppelbindung bedeuten und
worin die Alkylgruppen jeweils 1 - 4 C-Atome besitzen,
sowie von deren physiologisch unbedenklichen Säureadditionssalzen,
dadurch gekennzeichnet, daß man eine Verbindung der allgemeinen Formel II

Ind-A-$X^1$ II

worin

$X^1$ X oder $NH_2$ und

X Cl, Br, J, OH oder eine reaktionsfähig funktionell abgewandelte OH-Gruppe bedeuten und

Ind und A die angegebenen Bedeutungen

haben,

mit einer Verbindung der allgemeinen Formel III

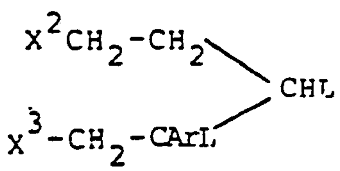

III

worin

$X^2$ und $X^3$ gleich oder verschieden sein können und, falls $X^1 = NH_2$ ist, jeweils X, andernfalls zusammen NH bedeuten und

Ar und L die angegebenen Bedeutungen haben,

umsetzt

oder daß man eine sonst der Formel I entsprechende Verbindung, die jedoch an Stelle eines oder mehrerer Wasserstoffatome eine oder mehrere reduzierbare Gruppe(n) und/oder eine oder mehrere zusätzliche C-C- und/oder C-N-Bindung(en) enthält, mit einem reduzierenden Mittel behandelt

oder daß man eine sonst der Formel I entsprechende Verbindung, die jedoch an Stelle eines oder mehrerer Wasserstoffatome eine oder mehrere solvolytisch abspaltbare Gruppe(n) enthält,

mit einem solvolysierenden Mittel behandelt oder daß man zur Herstellung von Verbindungen der Formel I worin

die beiden Reste L zusammen eine C-C-Bindung bedeuten, eine Verbindung der Formel IV

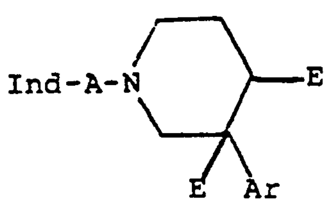

IV

worin

| | |
|---|---|
| der eine Rest | X, CN oder $NH_2$ |
| der andere Rest E | H bedeutet und |
| Ind, A, Ar und X | die angegebenen Bedeutungen haben |

mit einem HE-abspaltenden Mittel behandelt

und/oder daß man gegebenenfalls in einer Verbindung der Formel I eine Thioethergruppe zu einer SO-Gruppe oder $SO_2$-Gruppe oder eine SO-Gruppe zu einer $SO_2$-Gruppe oxydiert und/oder eine Alkoxygruppe unter Bildung einer OH-Gruppe spaltet und/oder daß man eine erhaltene Base der Formel I durch Behandeln mit einer Säure in eines ihrer physiologisch unbedenklichen Säureadditionssalze umwandelt.

4. Verfahren zur Herstellung pharmazeutischen Zubereitungen, dadurch gekennzeichnet, daß man eine Verbindung der Formel I nach Patentanspruch 1 und/oder eines ihrer physiologisch unbedenklichen Säureadditionssalze zusammen mit mindestens einem festen, flüssigen oder halbflüssigen Träger- oder Hilfsstoff und gegebenenfalls in Kombination mit einem oder mehreren weiteren Wirkstoffen in eine geeignete Dosierungsform bringt.

5. Pharmazeutische Zubereitung, gekennzeichnet durch einen Gehalt an mindestens einer Verbindung der allgemeinen Formel I nach Patentanspruch 1 und/oder einem ihrer physiologisch unbedenklichen Säureadditionssalze.

6. Verbindungen der allgemeinen Formel I nach Patentanspruch 1 zur Bekämpfung von Krankheiten.

7. Verwendung von Verbindungen der allgemeinen Formel I nach Patentanspruch 1 zur Herstellung pharmazeutlischer Zubereitungen.

**Claims**

1. Indole derivatives of the general formula I

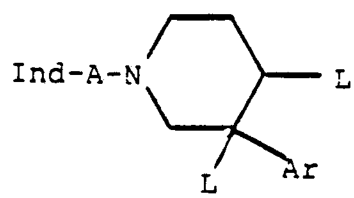

I

in which

Ind is a 3-indolyl radical which can be substituted once or twice by alkyl, O-alkyl, S-alkyl, SO-alkyl, $SO_2$-alkyl, OH, F, Cl, Br, $CF_3$ and/or CN or by a methylenedioxy group,

A is $-(CH_2)_n-$,

n is 3, 4 or 5,

Ar is a hydroxyphenyl group and

the two radicals L are each H or together are a C-C bond,

and in which the alkyl groups each have 1-4 C atoms,

and their physiologically acceptable acid addition salts.

2. 3-[4-(3-m-Hydroxyphenyl-1-piperidyl)butyl]indole.

3. Process for the preparation of indole derivatives of the general formula I

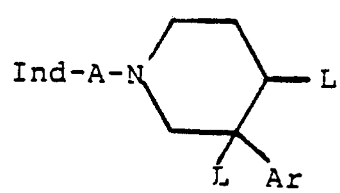

I

in which

Ind is a 3-indolyl radical which can be

substituted once or twice by alkyl, O-alkyl, S-alkyl, SO-alkyl, $SO_2$-alkyl, OH, F, Cl, Br, $CF_3$ and/or CH or by a methylenedioxy group,

A is $-(CH_2)_n-$,

n is 3, 4 or 5,

Ar is a hydroxyphenyl group and

the two radicals L are each H or together are a double bond,

and in which the alkyl groups each have 1 - 4 C atoms, and of their physiologically acceptable addition salts, characterised in that a compound of the general formula II

Ind-A-$X^1$ II

in which

$X^1$ is X or $NH_2$, and

X is Cl, Br, I, OH or a reactive derivative of an OH group, and

Ind and A have the indicated meanings,

is reacted with a compound of the general formula III

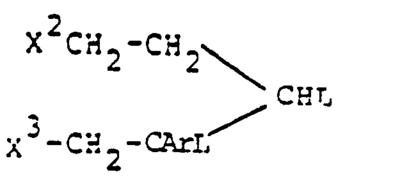

III

in which

$X^2$ and $X^3$ can be identical or different and, when $X^1$ is $NH_2$, are each X, but otherwise together are NH, and

Ar and L have the indicated meanings,

or in that a compound which otherwise corresponds to formula I, but contains one or more reducible group(s) and/or one or more additional C-C and/or C-N bond(s) in place of one or more hydrogen atoms, is treated with a reducing agent,

or in that a compound which otherwise corresponds to formula I, but contains one or more group(s) which can be eliminated by solvolysis in place of one or more hydrogen atoms, is treated with a solvolysing agent, or in that, to prepare compounds of the formula I in which the two radicals L are together a C-C bond, a compound of the formula IV

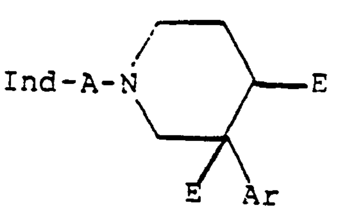

IV

in which

one radical E is X, CN or $NH_2$,

the other radical E is H, and

Ind, A, Ar and X have the indicated meanings, is treated with an agent which eliminates HE, and/or in that, where appropriate, in a compound of the formula I, a thioether group is oxidised to an SO group or $SO_2$ group, or an SO group is oxidised to an $SO_2$ group and/or an alkoxy group is cleaved with the formation of an OH group, and/or in that a base of the formula I obtained is converted, by treatment with an acid, into one of its physiologically acceptable acid addition salts.

4. Process for the preparation of pharmaceutical formulations, characterised in that a compound of the formula I according to Patent Claim 1 and/or one of its physiologically acceptable acid addition salts is converted into a suitable dosage form together with at least one solid, liquid or semiliquid vehicle or auxiliary and optionally in combination with one or more other active compounds.

5. Pharmaceutical formulation characterised by a content of at least one compound of the general formula I according to Patent Claim 1. and/or one of its physiologically acceptable acid addition salts.

6. Compounds of the general formula I according to Patent Claim 1 for controlling diseases.

7. Use of compounds of the general formula I according to Patent Claim 1 for the manufacture of pharmaceutical formulatios.

**Revendications**

1. Dérivés de l'indole répondant à la formule générale I

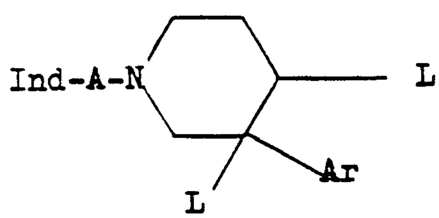

I

dans laquelle

Ind représente un groupe indole-3-yle qui peut être mono- ou di-substitué par des groupes alkyle, O-alkyle, S-alkyle, SO-alkyle, $SO_2$-alkyle, OH, F, Cl, Br, $CF_3$ et/ou CN ou par un groupe méthylènedioxy,

A représente $-(CH_2)_n-$,

n est égal à 3, 4 ou 5,

Ar représente un groupe hydroxyphényle, et

les deux symboles L représentent chacun H ou, ensemble, une liaison C-C et les groupes alkyle contiennent chacun 1 à 4 atomes de carbone,

et leurs sels acceptables pour l'usage pharmaceutique formés par addition avec des acides.

2. Le 3-[4-(3-m-hydroxyphényl-1-pipéridyl)-butyl]-indole.

3-. Procédé de préparation des dérivés de l'indole de formule générale I

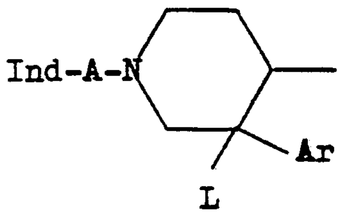

I

dans laquelle

Ind représente un groupe indole-3-yle qui peut être mono- ou di-substitué par des groupes alkyle, O-alkyle, S-alkyle, SO-alkyle, $SO_2$-alkyle, OH, F, Cl, Br, $CF_3$ et/ou CN ou par un groupe méthylènedioxy,

A représente $-(CH_2)_n-$,

n est égal à 3, 4 ou 5,

Ar représente un groupe hydroxyphényle, et

les deux symboles L représentent chacun H, ou ensemble, une double liaison, et les groupes alkyle contiennent chacun 1 à 4 atomes de carbone,

et de leurs sels acceptables pour l'usage pharmaceutique formés par addition avec des acides, caractérisé en ce que l'on fait réagir un composé de formule générale II

Ind-A-$X^1$ II

dans laquelle

$X^1$ représente X ou $NH_2$, et

X représente Cl, Br, I, OH ou un groupe OH ayant subi une modification fonctionnelle et réactif, et

Ind et A ont les significations indiquées ci-dessus,

avec un composé de formule générale III

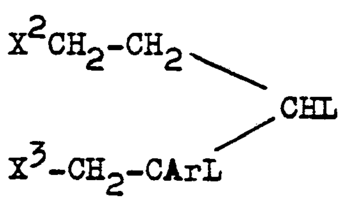

III

dans laquelle

$X^2$ et $X^3$, qui peuvent avoir des significations identiques ou différentes, représentent chacun X lorsque $X^1 = NH_2$ et dans les autres cas forment ensemble un groupe NH, et

Ar et L ont les significations indiquées ci-dessus, ou bien on part d'un composé répondant par ailleurs à la formule I mais contenant, à la place d'un ou plusieurs atomes d'hydrogène, un ou plusieurs groupes réductibles et/ou une ou plusieurs liaisons supplémentaires C-C et/ou C-N,

et on le traite par un agent réducteur,

ou bien on part d'un composé répondant par ailleurs à la formule I mais qui contient, à la place d'un ou plusieurs atomes d'hydrogène, un ou plusieurs groupes éliminables par solvolyse,

et on le traite par un agent solvolysant, ou bien, pour préparer les composés de formule I dans laquelle les deux symboles L représentent ensemble une liaison C-C, on traite un composé de formule IV

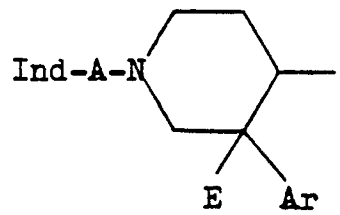

IV

dans laquelle

l'un des symboles E représente X, CN ou $NH_2$,

l'autre symbole E représente H, et

Ind, A, Ar et X ont les significations indiquées ci-dessus,

par un agent capable d'éliminer HE,

et/ou, le cas échéant, dans un composé de formule I, on oxyde un groupe thioéther en un groupe SO ou en un groupe $SO_2$ ou un groupe SO en groupe $SO_2$ et/ou on élimine un groupe alcoxy avec formation d'un groupe OH et/ou on convertit une base obtenue répondant à la formule I, en la traitant par un acide, en l'un de ses sels acceptables pour l'usage pharmaceutique formés par addition avec un acide.

4. Procédé de préparation de compositions pharmaceutiques, caractérisé en ce que l'on met un composé de formule I selon la revendication 1 et/ou l'un de ses sels acceptables pour l'usage pharmaceutique formés par addition avec un acide, avec au moins un véhicule ou produit auxiliaire solide, liquide ou semi-liquide et le cas échéant en combinaison avec une ou plusieurs autres substances actives, sous une forme de dosage appropriée.

5. Composition pharmaceutique caractérisée en ce qu'elle contient au moins un composé de formule générale I selon la revendication 1 et/ou l'un de ses sels acceptables pour l'usage pharmaceutique formés par addition avec un acide.

6. Composés de formule générale I selon la revendication 1, pour le traitement de maladies.

7. Utilisation des composés de formule générale I selon la revendication 1, pour la préparation de compositions pharmaceutiques.